Europäisches Patentamt

European Patent Office

Office européen des brevets

(19)

(11) **EP 1 330 562 B1**

(12) **EUROPEAN PATENT SPECIFICATION**

(45) Date of publication and mention
of the grant of the patent:
**25.05.2005 Bulletin 2005/21**

(51) Int Cl.[7]: **C30B 29/06**, C30B 15/00

(21) Application number: **01991951.3**

(86) International application number:
**PCT/US2001/044081**

(22) Date of filing: **22.10.2001**

(87) International publication number:
**WO 2003/004734 (16.01.2003 Gazette 2003/03)**

(54) **METHOD FOR THE PRODUCTION OF LOW DEFECT DENSITY SILICON**

VERFAHREN ZUR HERSTELLUNG VON SILICIUM MIT NIEDRIGER DEFEKTDICHTE

PROCEDE DE PRODUCTION D'UN SILICIUM A FAIBLE DENSITE DE DEFAUTS

(84) Designated Contracting States:
**DE FR GB IT**

(30) Priority: **03.11.2000 US 245610 P**
**22.11.2000 US 252715 P**
**31.05.2001 US 871255**
**05.10.2001 US 972608**

(43) Date of publication of application:
**30.07.2003 Bulletin 2003/31**

(73) Proprietor: **MEMC Electronic Materials, Inc.**
**St. Peters, Missouri 63376 (US)**

(72) Inventors:
• **VORONKOV, Vladimir**
**I-28100 Novara (IT)**
• **FALSTER, Robert J.**
**St. Peters, MO 63376 (US)**
• **BANAN, Mohsen**
**Grover, MO 63040 (US)**

(74) Representative: **Smaggasgale, Gillian Helen et al**
**W.P. Thompson & Co,**
**55 Drury Lane**
**London WC2B 5SQ (GB)**

(56) References cited:
**EP-A- 0 890 662       EP-A- 0 990 718**
**WO-A-98/45509        US-A- 5 919 302**

EP 1 330 562 B1

**Description**

BACKGROUND OF THE INVENTION

The present invention generally relates to the preparation of semiconductor grade single crystal silicon which is used in the manufacture of electronic components. More particularly, the present invention relates to a process for producing a single crystal silicon ingot by the Czochralski method, a substantial portion of which is substantially free of agglomerated intrinsic point defects, wherein the crystal growth conditions are varied to initially create alternating regions of silicon self-interstitial dominated material and vacancy dominated material within the constant diameter portion of the ingot. The regions of vacancy dominated material act as sinks to which self-interstitials may diffuse and be annihilated.

Single crystal silicon, which is the starting material for most processes for the fabrication of semiconductor electronic components, is commonly prepared by the so-called Czochralski ("Cz") method. In this method, polycrystalline silicon ("polysilicon") is charged to a crucible and melted, a seed crystal is brought into contact with the molten silicon and a single crystal is grown by slow extraction. After formation of a neck is complete, the diameter of the crystal is enlarged by decreasing the pulling rate and/or the melt temperature until the desired or target' diameter is reached. The cylindrical main body of the crystal which has an approximately constant diameter is then grown by controlling the growth rate and the melt temperature while compensating for the decreasing melt level. Near the end of the growth process but before the crucible is emptied of molten silicon, the crystal diameter is reduced gradually to form an end-cone. Typically, the end-cone is formed by increasing the crystal growth rate and heat supplied to the crucible. When the diameter becomes small enough, the crystal is then separated from the melt.

In recent years, it has been recognized that a number of defects in single crystal silicon form in the crystal growth chamber as the crystal cools after solidification. Such defects arise, in part, due to the presence of an excess (i.e. a concentration above the solubility limit) of intrinsic point defects, which are known as vacancies and self-interstitials. Silicon crystals grown from a melt are typically grown with an excess of one or the other type of intrinsic point defect, either crystal lattice vacancies ("V") or silicon self-interstitials ("I"). It has been suggested that the type and initial concentration of these point defects in the silicon are determined at the time of solidification and, if these concentrations reach a level of critical supersaturation in the system and the mobility of the point defects is sufficiently high, a reaction, or an agglomeration event, will likely occur. Agglomerated intrinsic point defects in silicon can severely impact the yield potential of the material in the production of complex and highly integrated circuits.

Vacancy-type defects are recognized to be the origin of such observable crystal defects as D-defects, Flow Pattern Defects (FPDs), Gate Oxide Integrity (GOI) Defects, Crystal Originated Particle (COP) Defects, crystal originated Light Point Defects (LPDs), as well as certain classes of bulk defects observed by infrared light scattering techniques such as Scanning Infrared Microscopy and Laser Scanning Tomography. Also present in regions of excess vacancies are defects which act as the nuclei for ring oxidation induced stacking faults (OISF). It is speculated that this particular defect is a high temperature nucleated oxygen agglomerate catalyzed by the presence of excess vacancies.

Defects relating to self-interstitials are less well studied. They are generally regarded as being low densities of interstitial-type dislocation loops or networks. Such defects are not responsible for gate oxide integrity failures, an important wafer performance criterion, but they are widely recognized to be the cause of other types of device failures usually associated with current leakage problems.

The density of such vacancy and self-interstitial agglomerated defects in Czochralski silicon is conventionally within the range of about $1*10^3/cm^3$ to about $1*10^7/cm^3$. While these values are relatively low, agglomerated intrinsic point defects are of rapidly increasing importance to device manufacturers and, in fact, are now seen as yield-limiting factors in device fabrication processes.

One approach which has been suggested to control the formation of agglomerated defects is to control the initial concentration of the point defects when the single crystal silicon is formed upon solidification from a molten silicon mass by controlling the growth rate (v) of the single crystal silicon ingot from the molten silicon mass, wherein higher growth rates tend to produce vacancy rich material and lower growth rates tend to produce interstitial rich material, and controlling the axial temperature gradient, G, in the vicinity of the solid-liquid interface of the growing crystal for a given temperature gradient. In particular, it has been suggested that the radial variation of the axial temperature gradient be no greater than 5 °C/cm. or less. See, e.g., Iida et al., EP0890662. This approach, however, requires rigorous design and control of the hot zone of a crystal puller.

Another approach which has been suggested to control the formation of agglomerated defects is to control the initial concentration of vacancy or interstitial point defects when the single crystal silicon is formed upon solidification from a molten silicon mass and controlling the cooling rate of the crystal from the temperature of solidification to a temperature of about 1,050 °C to permit the radial diffusion of silicon self-interstitial atoms or vacancies towards the lateral surface of the ingot or towards each other causing recombination thereby suppressing the concentration of intrinsic point defects to maintain the supersaturation of the vacancy system or the interstitial system at values which are less than those at which agglomeration reactions occur. See, for example, Falster et al., U.S. Patent No. 5,919,302 and Falster et al., WO 98/45509. While these approaches may be successfully used to prepare single crystal silicon which is substantially free of agglomerated vacancy or interstitial defects, significant time may be required to allow for adequate diffusion of vacancies and interstitials. This may have the effect of reducing the throughput for the crystal puller.

SUMMARY OF THE INVENTION

Among the several objects and features of the present invention may be noted a process for producing a single crystal silicon ingot, a significant portion of which is substantially free of agglomerated intrinsic point defects which negatively impact the semiconductor properties of the silicon; the provision of a process for producing a single crystal silicon ingot having alternating regions of vacancy dominated and silicon self-interstitial dominated regions said silicon self-interstitial dominated regions being substantially free of agglomerated intrinsic point defects; the provision of such a process which does not substantially diminish the throughput of the crystal puller; the provision of such a process which substantially reduces the need to limit growth rate in crystal puller; the provision of such a process which substantially reduces the need to limit the average axial temperature gradient $G_0$ in the crystal puller; and the provision of a method for sorting the wafers sliced from said single crystal silicon ingot such that wafers which are substantially free of agglomerated intrinsic defects may be visually identified.

Briefly, therefore, the present invention is directed to a process for the preparation of a silicon single crystal ingot, a substantial portion of which is substantially free of agglomerated intrinsic point defects. The process comprising controlling a growth velocity, v, and an average axial temperature gradient, $G_0$, such that the ratio of $v/G_0$ is varied to initially produce in the constant diameter portion of the ingot two or more regions, in which crystal lattice vacancies are the predominant intrinsic point defect, separated along the axis by one or more regions, in which silicon self-interstitial atoms are the predominant intrinsic point defect. The regions in which crystal lattice vacancies are initially the predominant intrinsic point defects have an axial length, $L_{vac.}$, and a radius, $R_{vac.}$, extending from the axis of the ingot towards the lateral surface which is at least about 10 % of the radius, R, of the constant diameter portion of the crystal. The region(s) in which silicon self-interstitial atoms are initially the predominant intrinsic point defects have an axial length of $L_{int.}$ and extend across the entire radius, R, of the constant diameter portion of the silicon single crystal. The regions are cooled from the temperature of solidification at a rate which allows silicon self-interstitial atoms and vacancies from the regions to diffuse and recombine to reduce the concentration of silicon self-interstitial atoms in the region(s) in which silicon self-interstitial atoms were initially the predominant intrinsic point defect and to reduce the concentration of crystal lattice vacancies in the region(s) in which crystal lattice vacancies were initially the predominant intrinsic point defect.

The present invention is further directed to a process for the preparation of a silicon single crystal ingot, a substantial portion of which is substantially free of agglomerated intrinsic point defects. The process comprising controlling a growth velocity, v, to initially produce in the constant diameter portion of the ingot two or more regions, in which crystal lattice vacancies are the predominant intrinsic point defect, separated along the axis by one or more regions, in which silicon self-interstitial atoms are the predominant intrinsic point defect. The regions in which crystal lattice vacancies are initially the predominant intrinsic point defects have an axial length, $L_{vac.}$, and a radius, $R_{vac.}$, extending from the axis of the ingot towards the lateral surface which is at least about 10 % of the radius, R, of the constant diameter portion of the crystal. The region(s) in which silicon self-interstitial atoms are initially the predominant intrinsic point defects have an axial length of $L_{int.}$ and extend across the entire radius, R, of the constant diameter portion of the silicon single crystal. The regions are cooled from the temperature of solidification at a rate which allows silicon self-interstitial atoms and vacancies from the regions to diffuse and recombine to reduce the concentration of silicon self-interstitial atoms in the region(s) in which silicon self-interstitial atoms were initially the predominant intrinsic point defect and to reduce the concentration of crystal lattice vacancies in the region(s) in which crystal lattice vacancies were initially the predominant intrinsic point defect.

The present invention is further directed to a single crystal silicon ingot having a constant diameter portion comprising multiple axially symmetric regions alternating along the axis of the ingot between a region wherein vacancies are the predominant intrinsic point defect and a region wherein interstitials are the predominant intrinsic point defect, said ingot having at least 2 interstitial dominant regions which are substantially free of agglomerated interstitial defects separated by a vacancy dominant region along the axis of the constant diameter portion of the ingot.

Other objects and features of this invention will be in part apparent and in part pointed out hereinafter.

BRIEF DESCRIPTION OF THE DRAWINGS

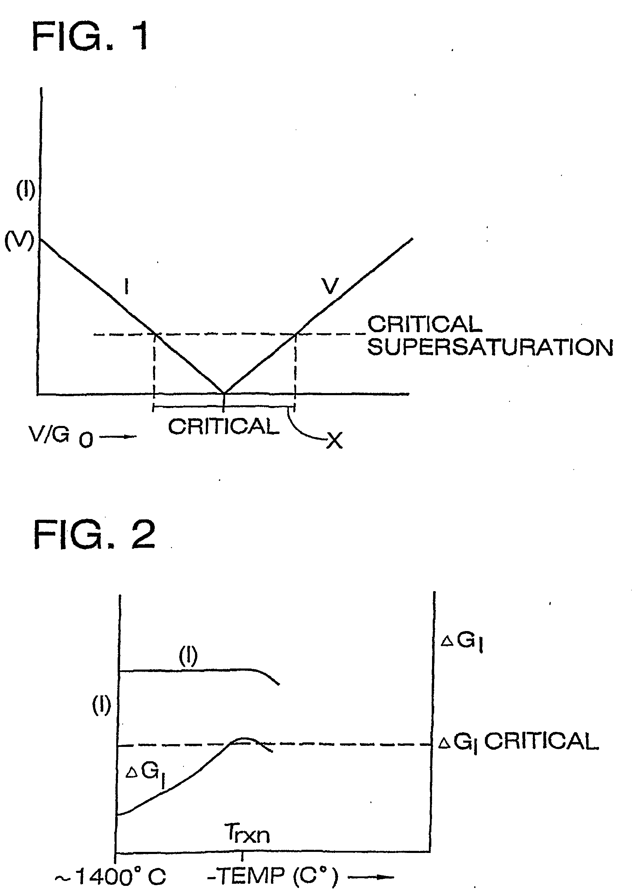

FIG. 1 is a graph which shows an example of how the initial concentration of self-interstitials, [I], and vacancies, [V], changes with an increase in the value of the ratio $v/G_0$, where v is the growth velocity and $G_0$ is the average axial temperature gradient.

FIG. 2 is a graph which shows an example of how $\Delta G_I$, the change in free energy required for the formation of agglomerated interstitial defects, increases as the temperature, T, decreases, for a given initial concentration of self-interstitials, [I].

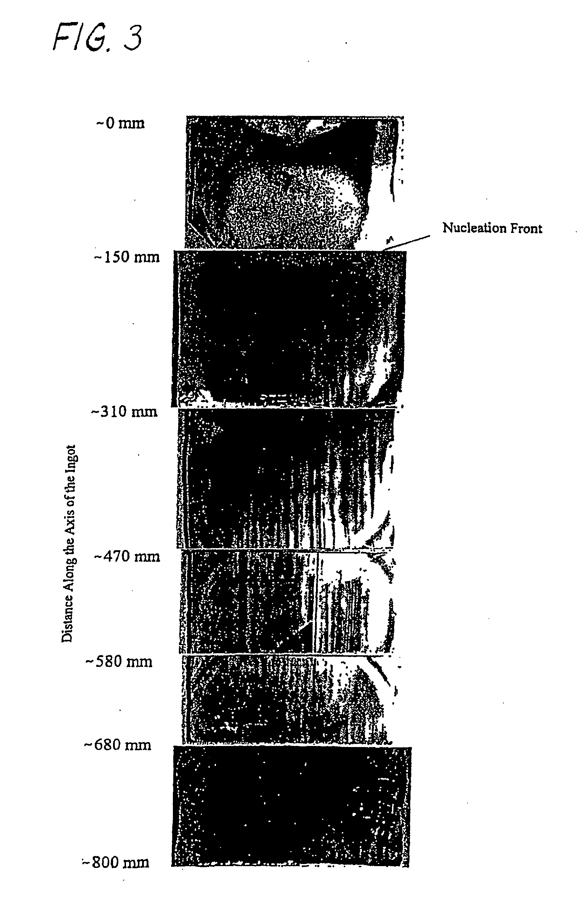

FIG. 3 is a cross-sectional image of an ingot prepared by quench cooling the ingot through the temperature range at which agglomerated intrinsic point defects nucleate.

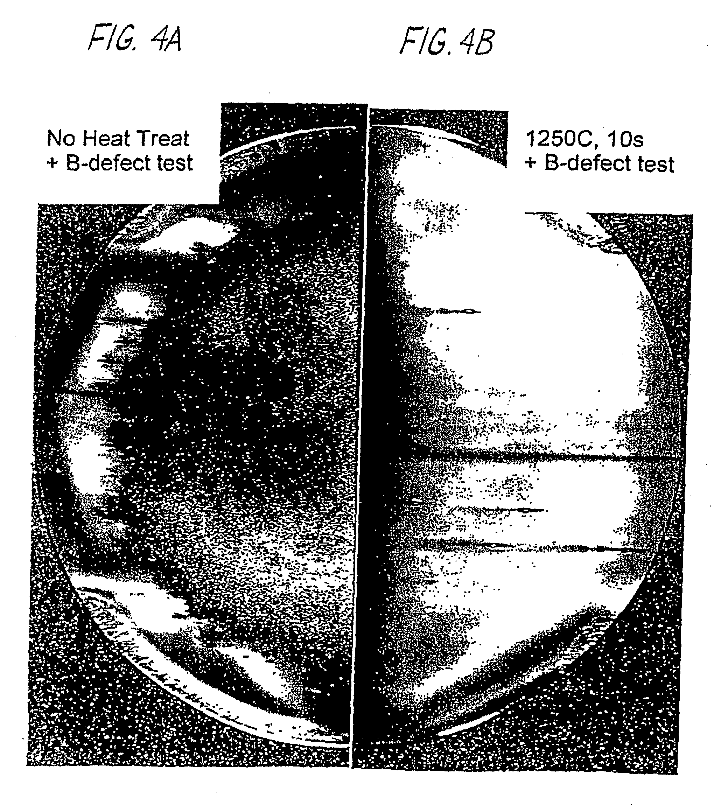

FIG. 4 is an image comparing a wafer having B-defects before being subjected to a B-defect annihilation heat-treatment to a wafer having B-defects which was subjected to a B-defect annihilation heat-treatment.

DETAILED DESCRIPTION OF THE PREFERRED EMBODIMENTS

Based upon experimental evidence to date, the type and initial concentration of intrinsic point defects appears to be initially determined as the ingot cools from the temperature of solidification (i.e., about 1410 °C) to a temperature greater than 1300 °C (i.e., at least about 1325 °C, at least about 1350 °C or even at least about 1375 °C). That is, the type and initial concentration of these defects are controlled by the ratio $v/G_0$, where v is the growth velocity and $G_0$ is the average axial temperature gradient over this temperature range.

The transition between vacancy and interstitial dominated material occurs at a critical value of $v/G_0$ which, based upon currently available information, appears to be about $2.1 \times 10^{-5}$ cm$^2$/sK where $G_0$ is determined under conditions in which the axial temperature gradient is constant within the temperature range defined above. At this critical value, the resulting concentrations of these intrinsic point defects are equal. If the value of $v/G_0$ exceeds the critical value, vacancies are the predominant intrinsic point defect and the concentration of vacancies increases with increasing $v/G_0$. If the value of $v/G_0$ is less than the critical value, silicon self-interstitials are the predominant intrinsic point defect and the concentration of silicon self-interstitials increases with decreasing $v/G_0$.

The ratio of $v/G_0$, and therefore the type of intrinsic point defect initially predominant in a particular region may be controlled by controlling the temperature gradient, $G_0$, the growth rate v, or by controlling both $G_0$ and v. Preferably, however, the ratio of $v/G_0$ is controlled by controlling the growth rate v. For a given $G_0$, therefore, a decrease in the growth rate, v, tends to increase the concentration of silicon self-interstitials and an increase in the growth rate, v, tends to increase the concentration of vacancies.

Once the initial concentration of an intrinsic point defects is established, the formation of agglomerated defects is thought to be dependent upon the free energy of the system. For a given concentration of intrinsic point defects a decrease in temperature yields an increase in the change in free energy for the reaction which forms agglomerated defects from the intrinsic point defects. Thus, as a region containing a concentration of vacancies or interstitials cools from the temperature of solidification through the temperature at which agglomerated defects are nucleated, the energy barrier for the formation of agglomerated vacancy or interstitial defects is approached. As cooling continues, this energy barrier may eventually be exceeded, at which point an agglomeration reaction occurs (*See*, e.g., Falster et al., U.S. Patent No. 5,919,302 and Falster et al., WO 98/45509).

The temperature at which nucleation of agglomerated defects occurs, i.e. the temperature of agglomeration, $T_A$, is dependent upon the concentration and type of predominant intrinsic point defects (vacancy or silicon self-interstitial). In general, the temperature of agglomeration, $T_A$, increases with increasing concentration of intrinsic point defect. In addition, the range of nucleation temperatures for agglomerated vacancy-type defects is somewhat greater than the range of nucleation temperatures for agglomerated interstitial-type defects; stated another way, over the range of vacancy concentrations typically produced in Czochralski grown single crystal silicon the temperature of agglomeration, $T_A$, for agglomerated vacancy defects is generally between about 1,000 °C and about 1,200 °C and typically between about 1,000 °C and about 1,100 °C whereas over the range of silicon self-interstitial concentrations typically produced in Czochralski grown single crystal silicon, the temperature of agglomeration, $T_A$, is generally between about 850 °C and about 1,100 °C and typically between about 870 °C and about 970 °C.

The concentration of vacancies or interstitials may be suppressed, however by allowing the ingot to remain above the temperature of agglomeration, $T_A$, for a time period sufficient to allow the vacancies or interstitials to diffuse radially to the surface of the ingot. However, large diameter ingots require substantial time periods for vacancies or interstitials located at the axis of the ingot to diffuse to the surface.

Ingots comprising concentrically positioned regions of vacancies and regions of interstitials, provide an additional mechanism for reducing the concentration of vacancies and interstitials, wherein the ingot may be maintained above the temperature of agglomeration, $T_A$, for a time period sufficient to allow the vacancies and interstitials to diffuse radially towards each other such that they recombine and are mutually annihilated.

In accordance with the present invention, at least a significant fraction of the constant diameter portion of the ingot may be grown substantially free of agglomerated intrinsic point defects by controlling the growth conditions to initially produce in the constant diameter portion of the ingot, separated regions along the axis having alternating predominant intrinsic point defects. Stated another way, the predominant intrinsic point defect alternates between vacancy dominated regions and silicon self-interstitial dominated regions along the axis of the constant diameter portion of the ingot. As a result, vacancies and interstitials located near the axis may diffuse axially towards each other and recombine while intrinsic point defects near the lateral surface can diffuse radially towards the surface, thus utilizing both radial and axial diffusion to suppress the concentration of intrinsic point defects. Because interstitials diffuse at much faster rates than vacancies, the interstitials will travel a much further distance than the vacancies prior to the recombination. As a practical matter, therefore, it is preferred that the constant diameter portion of the ingot initially comprise relatively larger regions of silicon self-interstitial dominated regions separated by relatively smaller vacancy dominated regions having a high concentration of vacancies. The vacancy dominated regions thus serve as sinks for the rapidly diffusing silicon self interstitials where they are annihilated to suppress the concentration of silicon self-interstitials prior to cooling below the temperature of agglomeration.

Without being held to a particular theory, it is believed that the out-diffusion of silicon self interstitials from an interstitial dominated region located axially between two vacancy dominated regions may be described by modeling the concentration field of interstitials. Accordingly, the concentration of interstitials can be described as a function of time by expanding the interstitial concentration field $C_i(r,z,t)$ into a series of eigenfunctions wherein each such function is a product of the Bessel function $J_o(\lambda r/R)$ for the radial concentration profile and of the sinusoidal function $\sin(\mu z/L)$ for the axial concentration profile and wherein $\lambda$ and $\mu$ are the roots of those two functions, at a distance (r) equal to the radius (R) of the region, and at a distance (z) along the axis equal to the length (L) of the region. Deep decay in the concentration implies that only the first term of the series is relevant such that:

$$C_i/C_m = A\, J_o(\lambda r/R)\, \sin(\pi z/L)\, \exp(-t/\tau) \qquad (2)$$

with $\lambda=2.4048$, wherein $C_m$ is the melting point equilibrium concentration and the ratio $C_i/C_m$ represents the normalized interstitial concentration. The decay time $\tau$ is expressed through the two eigenvalues, $\lambda/R$ and $\pi/L$ such that:

$$1/\tau = D\, [(\lambda/R)^2 + (\pi/L)^2] \qquad (3)$$

wherein D is the diffusion coefficient for silicon-self interstitials.

The expansion coefficient A in equation (2) is expressed through the imprinted interstitial concentration $C_{imp}=B(1-V/V_{cr})$, where B is about 0.5 such that:

$$A = 2.04\, B\, (1-V/V_{cr}) \qquad (4)$$

where the coefficient 2.04 is the product of the radial expansion coefficient 1.602 and the axial expansion coefficient $4/\pi=1.27$, v is the growth velocity and $V_{cr}$ is the critical growth velocity.

Furthermore, a relationship exists between the growth velocity, the time the ingot dwells above the temperature of agglomeration, $T_A$, and the dwell length, $L_{dw}$, corresponding to the distance over which a given axial position travels as the ingot cools from near the solidification temperature to $T_A$. The relationship between this distance, or "dwell length" ($L_{dw}$), the growth velocity, v, and the dwell time, $t_{dw}$, is expressed as follows:

$$t_{dw} = L_{dw}/v. \qquad (5)$$

To prevent the formation of agglomerated intrinsic point defects, the dwell time, $t_{dw}$, is preferably of sufficient duration to allow enough interstitials or vacancies to diffuse and be annihilated to sufficiently suppress the concentration thus preventing the formation of agglomerated intrinsic point defects as that portion of the ingot exceeds the dwell length and cools below the temperature of agglomeration.

The temperature profile in the hot zone of the crystal grower is believed to be almost unaffected by variations in the growth velocity, v, such that the dwell length, $L_{dw}$, may be considered as a constant for a given hot zone. Thus, the hot zone can be designed to provide the dwell length required to provide a sufficient dwell time based on a desired set of growth conditions, for example, the crystal diameter and growth rate.

Under steady growth processes, (ie. processes wherein the growth rate is controlled to produce an ingot which is predominantly interstitial rich or vacancy rich from center to edge throughout the growth of the ingot) there exists a relationship between the dwell length, $L_{dw}$, the critical growth rate, $V_{cr}$, and the radius of the ingot, R, wherein for a given ingot radius and critical growth rate, there exists a dwell length that will provide sufficient dwell time, $t_{dw}$, above the agglomeration temperature for the intrinsic point defects to diffuse to the surface of the ingot, suppressing the concentration and preventing the formation of agglomerated intrinsic point defects. The relationship is expressed as follows:

$$L_{dw} = 0.35 V_{cr} R^2 \qquad (6)$$

The same requirement is applicable to ingots having a segment wherein the predominant intrinsic point defect alternates along the axis of the ingot between regions wherein vacancies are the predominant intrinsic point defect and regions wherein silicon self-interstitials are the predominant intrinsic point defect. Provided the quantity of vacancies in the vacancy dominated regions is at least about the quantity of interstitials in the interstitial dominated region to allow for recombination, there will exist an additional mechanism for suppressing the concentration, i.e., axial diffusion and recombination. Accordingly, the concentration of interstitials will be suppressed faster than an ingot relying predominantly on radial diffusion. Stated differently, silicon-self interstitials in an interstitial dominated region having a radius equal to the radius, R, of the ingot and having vacancy dominated regions located along the axis of the ingot before and after the interstitial dominated region, will diffuse from the region both axially and radially causing the concentration of silicon-self interstitials to be suppressed at a faster rate. That is, because the silicon-self interstitials near the axis can diffuse axially to the vacancy dominated regions, the maximum length in which any silicon-self interstitial is required to travel before recombining and being eliminated is significantly reduced. To describe the diffusion rates and suppression of the interstitial concentration, the region can be characterized as having an effective radius, $R_{eff}$, less than the actual radius, R of the ingot. The effective radius, $R_{eff}$, is generally less than the crystal radius and may be expressed as a function of the ingot radius, R, and the axial length of the interstitial dominated region, bound along the axis in the core region radially near and including the axis, by vacancy dominated regions, as follows:

$$R_{eff} = R / [1 + (\pi R/\lambda L)^2]^{1/2} \qquad (7)$$

Thus, by substituting the effective radius, $R_{eff}$, for R in equation (6), the required dwell length to prevent the agglomeration of intrinsic point defects may be determined. For a considerable gain in the growth and cooling rate therefore, the length, $L_{int.}$, of an interstitial region, having a vacancy region both before and after the interstitial region along the axis of the ingot, is preferably comparable to about twice the radius R (or less), such that $R_{eff}$ becomes less than R, thus reducing the required dwell length according to equations (6) and (7) and correspondingly reducing the required dwell time, $t_{dw}$, according to equation (5). Thus, an effective dwell time, $t_{dw-eff.}$, can be achieved which is less than about 85%, less than about 60%, less than about 40%, and even less than about 20% of the dwell time that would otherwise be required if the silicon self-interstitial atoms were to diffuse primarily in the radial direction without the benefit of axial diffusion to suppress the concentration of silicon self-interstitial atoms.

To prevent the formation of agglomerated intrinsic point defects in interstitial dominant regions, the normalized concentration of interstitials, $C_i/C_m$ is preferably suppressed below some critical value, $(C_i/C_m)_{cr}$ (equal to about 0.01 at $T_A=920°C$). It follows from equations (2) through (4), that to suppress $C_i/C_m$ below the critical level of $(C_i/C_m)_{cr}=0.01$ (at $T_A=920°C$) the dwell time $t_{dw}$ is preferably long enough, to fulfill the condition:

$$Dt_{dw} [(\lambda/R)^2 + (p/L)^2] > \log[(1-V/V_{cr})/(C_i/C_m)_{cr}]. \qquad (8)$$

Accordingly, if the dwell time is specified (by a particular hot zone) then the required value of the length, $L_{int.}$, of the silicon self-interstitial dominant region may be determined from equation (8) for a growth rate ratio, $V/V_{cr}$, producing a silicon self-interstitial dominant region. For example, given a $V/V_{cr}$ ratio of about 0.9, the right-hand part of equation (8) becomes about 1. Therefore, by knowing the radius, R, of the ingot and the dwell time, $t_{dw}$, of the crystal grower, the length, $L_{int.}$, of an interstitial dominant region may be determined such that the concentration of interstitials will be suppressed below the critical level of $(C_i/C_m)_{cr}=0.01$ (at $T_A=920°C$) within the dwell time specified by the hot zone.

Ideally, the initial concentrations of vacancies in the vacancy dominant regions are sufficient to recombine with the silicon self interstitials, with the resulting vacancy concentrations being sufficiently suppressed to prevent agglomerated vacancies from forming as the ingot cools. However, because the vacancies diffuse at rates substantially slower than silicon self-interstitials, as a practical matter, the concentration of the vacancy dominated region may not be suppressed sufficiently to prevent agglomerated vacancies. Furthermore, it is preferable that the vacancy dominated regions contain an excess of vacancies over the quantity of vacancies required to combine with and annihilate silicon self interstitials to suppress the concentration of silicon self-interstitials. This excess may result in agglomerated vacancies upon cooling below the agglomeration temperature.

The concentrations of vacancies and interstitials in each region are proportional to the growth rate ratios, $(V/V_{cr})_{vacancy}$ and $(V/V_{cr})_{interstitial}$. Accordingly, the minimum length ratio for the vacancy dominated region to the interstitial dominated region required to provide a quantity of vacancies equal to the number of silicon self interstitials, may be expressed in relation to the normalized growth rate ratio such that:

$$[L_{vacancy}/L_{interstitial}]_{min} = 0.37\ [(V/V_{cr})_{interstitial}/(V/V_{cr})_{vacancy}] \tag{9}$$

The growth rate during the formation of the vacancy regions may be any growth rate in excess of the critical growth rate without departing from the scope of the invention. It is to be noted, however; that as the growth rate decreases, approaching the critical growth rate, i.e. the closer $(V/V_{cr})_{vacancy}$ gets to 1, the concentration of vacancies and typically the radius of the vacancy dominated region decreases, decreasing the total quantity of vacancies available for recombination. According to equation (9), therefore, decreases in $(V/V_{cr})_{vacancy}$ cause increases in the required length of the vacancy dominated regions relative to the length of silicon self-interstitial region. Correspondingly, as the growth rate increases, the concentration of vacancies and typically the diameter of the vacancy dominated region increase, increasing the total quantity of vacancies available for recombination, thus decreasing the required length of the vacancy dominated regions relative to the length of silicon self-interstitial dominated region. Preferably, therefore, the growth velocity is controlled during the formation of the vacancy dominated regions, such that the ratio $(V/V_{cr})_{vacancy}$ is at least about 1.5, more preferably at least about 2.0, more preferably at least about 2.5 and may even be at least about 3.5 or greater depending on the particular hot zone design.

Conversely, as the growth velocity in the silicon self-interstitial region becomes closer to the critical growth velocity, i.e., the ratio $(V/V_{cr})_{interstitial}$ is increased, the concentration of silicon self-interstitials is reduced and, according to equation (9), $[L_{vac.}/L_{int.}]_{min}$ is reduced. Preferably, during the growth of the silicon self-interstitial region, the growth velocity is controlled such that the ratio $(V/V_{cr})_{interstitial}$ is from about 0.5 to about 0.95, more preferably from about 0.7 to about 0.9, and most preferably from about 0.8 to about 0.9. Although slower growth velocities may be utilized without departing from the scope of the invention, as a practical matter, reduced growth velocities decrease the throughput of the crystal growth process and therefore are not desirable. Additionally, as the growth velocity is decreased further below the critical growth velocity, the total quantity of silicon-self interstitials increases.

Preferably the vacancy dominated regions are as short as possible, with a greater total volume of the constant diameter portion of the ingot preferably being grown as silicon self-interstitial dominant material to improve the overall yield of substantially defect-free silicon. However, it is also preferred that the total number of vacancies is at least about as much as the total number of silicon self-interstitials. Thus, it is preferred that the growth velocity is cycled asymmetrically about the critical growth velocity. That is, it is preferred that the growth velocity is increased to at least about 150 percent and as much as 350 percent of the critical growth rate during the formation of the vacancy dominated region while preferably being reduced to only about 90 percent of the critical growth rate during the formation of the interstitial dominated region.

Because interstitials diffuse faster than vacancies, the interface between the interstitial dominant region and the vacancy dominated regions will shift causing the resulting interstitial dominant region, substantially free of agglomerated intrinsic point defects to have an axial length somewhat greater than $L_{int.}$. Preferably, the number of vacancies in the vacancy dominant region is in excess of the quantity of silicon self-interstitials in the interstitial dominant region. Accordingly, the length ratio, $[L_{vac.}/L_{int.}]$ may be selected at some value greater than the minimum for a given set of growth velocities to provide an excess of vacancies.

In general, a silicon single crystal may be grown by the Czochralski method to form an ingot having a central axis, a seed-cone, an end-cone, a constant diameter portion between the seed-cone and the end-cone having a lateral surface, and a radius, R, extending from the central axis to the lateral surface. The radius, R, of the constant diameter portion of the ingot is preferably at least about 75 mm, at least about 100 mm and may even be at least about 150 mm or greater and has an axial length, L, of at least about 400 mm, at least about 600 mm, and may even be at least about 1000 mm or greater.

In accordance with the present invention, the ratio of the growth velocity over the average axial temperature gradient, $v/G_0$, is controlled to initially produce in the constant diameter portion of the ingot two or more regions, in which crystal lattice vacancies are the predominant intrinsic point defect, separated along the axis by one or more regions, in which silicon self-interstitial atoms are the predominant intrinsic point defect by intentionally varying the ratio $v/G_0$. In a preferred embodiment, the ratio is controlled by varying the growth velocity as discussed above.

In particular, the growth velocity is controlled such that the region or regions in which silicon self-interstitial atoms are the predominant intrinsic point defect have an axial length of $L_{int.}$ which is less than twice the radius of the constant diameter portion of the ingot and has a radius $R_{int.}$ which extends across the entire radius, R, of the constant diameter portion of the ingot to take advantage of the axial diffusion and recombination of intrinsic point defects in the near axis region of the ingot. It is preferred that the ingot is grown under conditions in which a substantial portion of the ingot comprises a region or regions in which silicon self-interstitial atoms are the predominant intrinsic point defects. Preferably, therefore, the axial length of the interstitial dominant regions, $L_{int.}$, is at least about 25% of the radius of the constant diameter portion of the ingot.

Preferably, therefore, the axial length, $L_{int.}$, of the interstitial dominant region is at least about 25% and less than about 2R, at least about 50% and less about than about 1.5R, and preferably about equal to the radius, R, of the constant diameter portion of the ingot. The axial length, $L_{int.}$, of the interstitial dominant region may exceed 2R or be less than 25% of R, however regions having an axial length greater than 2R will have increased diffusion times required to suppress the concentration of interstitials relying increasingly on radial diffusion from the axis to the lateral surface, and regions having an axial length less than 25% may reduce the total volume of substantially defect free silicon in the ingot.

The growth rate is further controlled such that the regions in which crystal lattice vacancies are initially the predominant intrinsic point defects have a radius, $R_{vac.}$, extending from the axis of the ingot towards the lateral surface which is at least about 10%, preferably at least about 50%, and even more preferably at least about 90% or greater of the radius, R, of the constant diameter portion of the crystal. The initial axial length, $L_{vac.}$, of the vacancy dominated regions is preferably at least about 0.05 times the initial axial length, $L_{int.}$, of the interstitial dominated regions and may be at least about 0.1, or even at least about 0.5 times the initial axial length, $L_{int.}$, of the interstitial dominated regions, depending on the ratio between the normalized growth rate for the vacancy dominated regions and the normalized growth rate of the interstitial dominated regions as described in equation (9). For example, if $V_{vac.}$ is 1.5 times $V_{cr}$ and $V_{int.}$ is 0.9 times $V_{cr}$ then ($L_{vac.}/L_{int.}$) is preferably at least about 0.22. Similarly, if $V_{vac.}$ is 2 times $V_{cr}$ and $V_{int.}$ is 0.9 times $V_{cr}$ then ($L_{vac.}/L_{int.}$) is preferably at least about 0.17; if $V_{vac.}$ is 2.5 times $V_{cr}$ and $V_{int.}$ is 0.9 times $V_{cr}$ then ($L_{vac.}/L_{int.}$) is preferably at least about 0.14; if $V_{vac.}$ is 1.5 times $V_{cr}$ and $V_{int.}$ is 0.5 times $V_{cr}$ then ($L_{vac.}/L_{int.}$) is preferably at least about 0.12; if $V_{vac.}$ is 2 times $V_{cr}$ and $V_{int.}$ is 0.5 times $V_{cr}$ then ($L_{vac.}/L_{int.}$) is preferably at least about 0.1; and, if $V_{vac.}$ is 2.5 times $V_{cr}$ and $V_{int.}$ is 0.5 times $V_{cr}$ then ($L_{vac.}/L_{int.}$) is preferably at least about 0.08.

Thus, according to the process of the present invention, the growth rate is preferably controlled to initially produce in the constant diameter portion of the ingot at least about 2 vacancy dominated regions separated along the axial direction by at least 1 interstitial dominated region. More preferably, the growth rate is controlled to initially produce in the constant diameter portion of the ingot, at least about 4, 6, 10, and even as many as 16 or greater vacancy dominated regions separated along the axis by one or more interstitial dominated region(s).

As noted above, the radius of the vacancy dominated region(s) may be less than the radius of the constant diameter portion of the ingot. Additionally, vacancy dominated regions having a radius less than the radius of the constant diameter portion of the ingot, will generally have a silicon self-interstitial dominated region extending radially inward from the lateral surface of the ingot to the vacancy dominated region. Thus, multiple vacancy dominated regions may be separated along the axis of the ingot by a single continuous interstitial dominated region, having a radius that varies from a minimum approximately equal to the difference between the radius of the vacancy dominated region and the radius of the constant diameter portion of the ingot to a maximum equal to the radius of the constant diameter portion of the ingot. For example, several vacancy dominated regions each having a radius equal to 10% of the radius of the constant diameter portion of the ingot may be separated along the axis of the ingot by a single continuous interstitial dominated region, having a radius which varies between 90% and 100% of the radius of the constant diameter portion of the ingot, wherein the vacancy dominated regions are separated along the axis when the interstitial dominated region has a radius equal to 100% of the radius of the constant diameter portion of the ingot. Alternatively, multiple vacancy dominated regions having a radius equal to the radius of the constant diameter portion of the ingot may be separated along the axis of the ingot by multiple interstitial dominated regions each having a radius equal to the constant diameter portion of the ingot.

Therefore, according to the process of the present invention, the growth rate is preferably controlled to initially form in the ingot one or more interstitially dominated regions separated along the axis by the vacancy dominated regions referred to above, wherein the interstitial dominated region(s) may be discontinuous regions completely separated along the axis by multiple vacancy regions having a radius equal to the constant diameter portion of the ingot, or one or more continuous region (s) separated along the axis by multiple vacancy regions having a radius less than the radius of the constant diameter portion of the ingot. Thus, according to the present invention, the growth rate is controlled to initially form 1 or more interstitially dominated regions having at least one vacancy dominated region axially adjacent to the interstitially dominated region(s), the vacancy dominated regions serving as a sink to which silicon selfinterstitials near the axis may diffuse and be annihilated.

The interstitial dominated regions are then cooled from the temperature of solidification at a rate which allows silicon self-interstitial atoms and vacancies from the regions to diffuse both radially and axially such that a sufficient number of silicon self-interstitial atoms recombine with vacancies to reduce the concentration of silicon self-interstitial atoms in the region(s) in which silicon self-interstitial atoms were initially the predominant intrinsic point defect and to reduce the concentration of crystal lattice vacancies in the region(s) in which crystal lattice vacancies were initially the predominant intrinsic point defect. Preferably, the regions are maintained at a temperature above $T_A$ for a time period of at least an effective dwell time, $t_{dw-eff.}$, required to allow a sufficient quantity of silicon self-interstitial atoms to diffuse to the surface of the ingot and to the vacancy dominated region(s) to suppress the concentration of silicon self-interstitial atoms in the region(s) in which silicon self-interstitial atoms were initially the predominant intrinsic point defect below a critical concentration required for agglomerated intrinsic point defects to nucleate.

Once the concentration of interstitials in the region has been suppressed, the region may be cooled such that a significant portion of the resulting ingot is substantially free of any agglomerated intrinsic point defects.

Depending upon the resulting concentration and distribution of vacancies and self-interstitial atoms in the various regions of the ingot, the formation of agglomerated intrinsic point defects may thereafter be avoided by controlling the diffusion of the intrinsic point defects and/or by quench cooling the ingot. Thus, if the resulting concentration of vacancies or silicon self-interstitials is below the concentration at which vacancies or silicon self-interstitials may agglomerate upon cooling, the region may be allowed to cool by the standard Czochralski process. If, however the vacancy or interstitial dominated regions or portions thereof have vacancy or interstitial concentrations greater than the concentration at which the interstitials or vacancies may agglomerate upon cooling, the cooling rate of the region may be controlled such that additional time is allowed for silicon self-interstitials and/or vacancies to diffuse radially to the lateral surface of the ingot and/or diffuse towards and recombine with each other further suppressing the concentration of vacancies and/or silicon self-interstitials during cooling such that the resulting ingot is substantially free of agglomerated intrinsic point defects (See for example co-pending United States Patent Application Numbers 09/344,036 and 09/344,709, now granted as United States Patents Nos. 6,312,516 and 6,328,795 respectively.) Additionally, if the concentration of vacancies or silicon self-interstitials is greater than the concentration at which the vacancies or silicon self-interstitials may agglomerate upon cooling, the ingot or portions thereof may be quench cooled such that the vacancies or silicon self-interstitials are effectively frozen in position without allowing sufficient time for them to agglomerate, such that the resulting ingot is substantially free of agglomerated intrinsic point defects as described in United States Application Number 09/661,745 claiming priority from Provisional Application Number 60/155,725 now published as WO 00/25525.

Alternatively, the concentration of interstitials in the interstitial dominated region(s) may only be suppressed such that upon cooling, the region(s) contain some agglomerated interstitial type defects, wherein the agglomerated interstitial-type defects are B-type defects only.

In general, control of the average axial temperature gradient, $G_0$, may be achieved primarily through the design of the "hot zone" of the crystal puller, i.e. the graphite (or other materials) that makes up the heater, insulation, heat and radiation shields, among other things. Although the design particulars may vary depending upon the make and model of the crystal puller, in general, $G_0$ may be controlled using any of the means currently known in the art for controlling heat transfer at the melt/solid interface, including reflectors, radiation shields, purge tubes, light pipes, and heaters. In general, radial variations in $G_0$ are minimized by positioning such an apparatus within about one crystal diameter above the melt/solid interface. $G_0$ can be controlled further by adjusting the position of the apparatus relative to the melt and crystal. This is accomplished either by adjusting the position of the apparatus in the hot zone, or by adjusting the position of the melt surface in the hot zone. In addition, when a heater is employed, $G_0$ may be further controlled by adjusting the power supplied to the heater. Any, or all, of these methods can be used during a batch Czochralski process in which melt volume is depleted during the process.

Varying the growth rate during the growth of the portion of ingot typically referred to as the constant diameter portion, i.e. the portion between the seed cone and the end cone, typically causes variations in the diameter along the axis. That is, the diameter of vacancy dominated regions is less than the diameter of interstitial dominated regions. As such, wafers sliced from the ingot having reduced diameters will typically be vacancy dominated; wafers sliced from the ingot having increased diameters will typically be interstitial dominated. Accordingly, the diameter of the ingot and subsequently the diameter of wafers sliced from the ingot may be used to sort the resulting wafers between those wafers having vacancies as the predominant intrinsic point defect, and those wafers having silicon self-interstitials as the predominant intrinsic point defect.

Definitions

It is to be noted that, as used herein, the following phrases shall have the given meanings: "agglomerated intrinsic point defects" shall mean defects caused (i) by the reaction in which vacancies agglomerate or (ii) by the reaction in which self-interstitials agglomerate; "agglomerated vacancy defects" shall mean agglomerated vacancy point defects caused by the reaction in which crystal lattice vacancies agglomerate, examples include D-defects, flow pattern defects, gate oxide integrity defects, crystal originated particle defects, and crystal originated light point defects; "agglomerated interstitial defects" shall mean agglomerated intrinsic point defects caused by the reaction in which silicon self-interstitial atoms agglomerate to form A-defects (including dislocation loops and networks) and B-defects; "B-defects" shall mean agglomerated interstitial defects which are smaller than A-defects and which are capable of being dissolved if subjected to a heat treatment; "radius" shall mean the distance measured from a central axis to a circumferential edge of a single crystal silicon sample, such as a wafer, or an ingot slug or slab; "substantially free of agglomerated intrinsic point defects" shall mean a concentration of agglomerated defects which is less than the detection limit of these defects, which is currently about $10^4$ defects/$cm^3$; "vacancy dominated" and "self-interstitial dominated" shall mean material in which the intrinsic point defects are predominantly vacancies or self-interstitials, respectively; and "visual detection of agglomerated intrinsic point defects," as well as variations thereof, shall refer to the detection of such defects using the naked eye under ordinary incandescent or fluorescent light sources, or optionally collimated or other enhanced light sources, and without the use of any instrumentation which would otherwise aid in defect detection or result in defect magnification, such as optical or infrared microscopy, X-ray diffraction, or laser scattering.

Detection of Agglomerated Defects

Agglomerated defects may be detected by a number of different techniques. For example, flow pattern defects, or D-defects, are typically detected by preferentially etching the single crystal silicon sample in a Secco etch solution for about 30 minutes, and then subjecting the sample to microscopic inspection. (see, e.g., H. Yamagishi et al., Semicond. Sci. Technol. 7, A135 (1992)). Although standard for the detection of agglomerated vacancy defects, this process may also be used to detect A-defects. When this technique is used, such defects appear as large pits on the surface of the sample when present.

Additionally, agglomerated intrinsic point defects may be visually detected by decorating these defects with a metal capable of diffusing into the single crystal silicon matrix upon the application of heat. Specifically, single crystal silicon samples, such as wafers, slugs or slabs, may be visually inspected for the presence of such defects by first coating a surface of the sample with a composition containing a metal capable of decorating these defects, such as a concentrated solution of copper nitrate. The coated sample is then heated to a temperature between about 900°C and about 1000°C for about 5 minutes to about 15 minutes in order to diffuse the metal into the sample. The heat treated sample is then cooled to room temperature, thus causing the metal to become critically supersaturated and precipitate at sites within the sample matrix at which defects are present.

After cooling, the sample is first subjected to a non-defect delineating etch, in order to remove surface residue and precipitants, by treating the sample with a bright etch solution for about 8 to about 12 minutes. A typical bright etch solution comprises about 55 percent nitric acid (70% solution by weight), about 20 percent hydrofluoric acid (49% solution by weight), and about 25 percent hydrochloric acid (concentrated solution).

The sample is then rinsed with deionized water and subjected to a second etching step by immersing the sample in, or treating it with, a Secco or Wright etch solution for about 35 to about 55 minutes. Typically, the sample will be etched using a Secco etch solution comprising about a 1:2 ratio of 0.15 M potassium dichromate and hydrofluoric acid (49% solution by weight). This etching step acts to reveal, or delineate, agglomerated defects which may be present.

In an alternative embodiment of this "defect decoration" process, the single crystal silicon sample is subjected to a thermal anneal prior to the application of the metal-containing composition. Typically, the sample is heated to a temperature ranging from about 850 °C to about 950 °C for about 3 hours to about 5 hours. This embodiment is particularly preferred for purposes of detecting B-type silicon self-interstitial agglomerated defects. Without being held to a particular theory, it is generally believed that this thermal treatment acts to stabilize and grow B-defects, such that they may be more easily decorated and detected.

Agglomerated vacancy defects may also be detected using laser scattering techniques, such as laser scattering tomography, which typically have a lower defect density detection limit that other etching techniques.

In general, regions of interstitial and vacancy dominated material free of agglomerated defects can be distinguished from each other and from material containing agglomerated defects by the copper decoration technique described above. Regions of defect-free interstitial dominated material contain no decorated features revealed by the etching whereas regions of defect-free vacancy dominated material (prior to a high-temperature oxygen nuclei dissolution treatment as described above) contain small etch pits due to copper decoration of the oxygen nuclei.

In view of the above, it will be seen that the several objects of the invention are achieved. As various changes could be made in the above-described process without departing from the scope of the invention, it is intended that all matters contained in the above description be interpreted as illustrative and not in a limiting sense. In addition, when introducing elements of the present invention or the preferred embodiment(s) thereof, the articles "a," "an," "the" and "said" are intended to mean that there are one or more of the elements. The terms "comprising," "including" and "having" are intended to be inclusive and mean that there may be additional elements other than the listed elements.

**Claims**

1. A process for the preparation of a silicon single crystal in which molten silicon is solidified into a crystal in accordance with the Czochralski method to form an ingot having a central axis, a seed-cone, an end-cone, a constant diameter portion between the seed-cone and the end-cone having a lateral surface, and a radius, R, extending from the central axis to the lateral surface of at least about 75mm, the constant diameter portion having an axial length, L, the process comprising;

   controlling a ratio, $v/G_0$, wherein, v is a growth velocity and $G_0$ is an average axial temperature gradient over the temperature range from solidification to a temperature of no less than about 1325 °C, during the growth of the constant diameter portion of the crystal, to initially produce in the constant diameter portion of the ingot a series of predominant intrinsic point defects alternating along the axis, the series comprising $N_{vac.}$ vacancy dominated regions and $N_{int.}$ silicon self interstitial dominated regions, wherein $N_{vac}$ is at least 2 and $N_{int.}$ is at least 1, the vacancy dominated regions each having an axial length, $L_{vac.}$, and a radius, $R_{vac.}$, extending from the axis of the ingot towards the lateral surface which is at least about 10% of the radius, R, of the constant diameter portion of the crystal, the silicon self-interstitial dominated region(s) each having an axial length, $L_{int.}$ and a radial width which is equal to the radius, R, of the constant diameter portion of the silicon single crystal;

   cooling said regions from the temperature of solidification at a rate which allows silicon self-interstitial atoms to diffuse radially to the lateral surface and to diffuse axially to vacancy dominated regions to reduce the concentration of silicon self-interstitial atoms in the region(s) in which silicon self-interstitial atoms were initially the predominant intrinsic point defect and to reduce the concentration of crystal lattice vacancies in the region(s) in which crystal lattice vacancies were initially the predominant intrinsic point defect.

2. A process according to claim 1, wherein the ratio, $v/G_0$, is controlled by controlling the growth velocity, v.

3. A process according to claim 1 or claim 2, wherein the regions in which crystal lattice vacancies are the predominant intrinsic point defects have a radius, $R_{vac.}$, extending from the axis of the ingot towards the lateral surface which is at least about 50 % (preferably at least about 90 %) of the radius, R, of the constant diameter portion of the crystal.

4. A process according to any one of claims 1 to 3, wherein the cooling rate of the ingot is controlled such that the regions are cooled from the temperature of solidification to a temperature, $T_A$, at which agglomerated intrinsic point defects nucleate, such that the regions are maintained at a temperature above $T_A$ for a time period of at least an effective dwell time, $t_{dw-eff.}$, required to prevent the formation of agglomerated intrinsic point defects in the interstitial dominated regions.

5. A process according to claim 4, wherein the temperature, $T_A$, at which agglomerated interstitials nucleate is from about 850°C to about 1,100°C (preferably from about 870°C to about 970°C).

6. A process according to claim 2, wherein the growth velocity is controlled such that the growth velocity during the formation of the vacancy dominated region, $v_{vac.}$, is at least about 1.5 times a critical value, $v_{crit.}$, and the growth velocity during the formation of the interstitial dominated region, $v_{int.}$, is less than about 0.9 times the critical value $v_{crit.}$.

7. A process according to claim 6, wherein the ratio of $L_{vac.}/L_{int.}$ is at least about 0.23.

8. A process according to claim 6 or claim 7, wherein $v_{vac.}$ is at least, about 2 times the critical value, $v_{crit.}$, during the growth of the vacancy dominated regions.

9. A process according to claim 8, wherein the ratio of $L_{Vac.}/L_{int.}$ is at least about 0.17.

10. A process according to claim 6, wherein $V_{vac.}$ is at least about 2.5 times the critical value, $v_{crit.}$, during the growth of the vacancy dominated regions.

11. A process according to claim 10, wherein the ratio of $L_{vac.}/L_{int.}$ is at least about 0.14.

12. A process according to claim 2, wherein the growth velocity is controlled such that the growth velocity during the formation of the vacancy dominated region, $v_{vac.}$, is at least about 1.5 times a critical value, $v_{crit.}$, and the growth velocity during the formation of the interstitial dominated region, $v_{int.}$, is less than about 0.5 times the critical value $v_{crit.}$.

13. A process according to claim 12, wherein the ratio of $L_{vac.}/L_{int.}$ is at least about 0.12.

14. A process according to claim 12, wherein $v_{vac.}$ is at least about 2 times the critical value, $v_{crit.}$, during the growth of the vacancy dominated regions.

15. A process according to claim 14, wherein the ratio of $L_{vac.}/L_{int.}$ is at least about 0.1.

16. A process according to claim 12, wherein $v_{vac.}$ is at least about 2.5 times the critical value, $v_{crit.}$, during the growth of the vacancy dominated regions.

17. A process according to claim 16, wherein the ratio of $L_{vac.}/L_{int.}$ is at least about 0.08.

18. A process according to any one of claims 1 to 17, wherein region(s) in which silicon self-interstitial atoms are the predominant intrinsic point defects have an axial length, $L_{int.}$, which is at least about 25% of the radius, R, of the constant diameter portion of the crystal but preferably less than about twice the radius, R, of the constant diameter portion of the crystal, and even more preferably an axial length, $L_{int.}$, which is about equal to the radius, R, of the constant diameter portion of the crystal.

19. A process according to claim 18, wherein the effective dwell time, $t_{dw-eff.}$, is at least about 20% of the dwell time required to allow a sufficient quantity of silicon self-interstitial atoms to diffuse to the surface of the ingot only to suppress the concentration of silicon self-interstitial atoms below a critical concentration required for agglomerated intrinsic point defects to nucleate, in an ingot wherein silicon self-interstitial atoms are the predominant intrinsic point defect throughout the entire constant diameter portion of an ingot.

20. A process according to claim 19, wherein the effective dwell time, $t_{dw-eff.}$, is less than about 85% of the dwell time, preferably about 60% of the dwell time, required to allow a sufficient quantity of silicon self-interstitial atoms to diffuse to the surface of the ingot only to suppress the concentration of silicon self-interstitial atoms below a critical concentration required for agglomerated intrinsic point defects to nucleate, in an ingot wherein silicon self-interstitial atoms are the predominant intrinsic point defect throughout the entire constant diameter portion of an ingot.

21. A process according to any one of claims 1 to 20, wherein the radius of the constant diameter portion of the ingot, R, is at least about 100 mm, preferably at least about 150 mm.

22. A process according to any one of claims 1 to 21, wherein the length of the constant diameter portion of the ingot, L, is at least about 400 mm, for example, at least about 600 mm, or at least about 1000 mm.

23. A process according to any one of claims 1 to 22, wherein the constant diameter portion of the ingot initially comprises at least about 2 or 4 or 6 or 8 vacancy dominated regions.

24. A process according to any one of claim 1 to 23, wherein the total quantity of vacancies in the initial vacancy dominated regions is greater than the total quantity of interstitials in the initial interstitial dominated regions.

25. A process according to any one of claims 1 to 24, further comprising quench cooling the ingot to a temperature less than a temperature at which agglomerated intrinsic point defects nucleate.

26. A single crystal silicon ingot having a central axis, a seed-cone, an end-cone, and a constant diameter portion between the seed-cone and the end-cone having a circumferential edge and a radius extending from the central axis to the circumferential edge, the single crystal silicon ingot, after being grown and cooled from the solidification temperature, having a constant diameter portion comprising multiple axially symmetric regions alternating along the axis of the ingot between a region wherein vacancies are the predominant intrinsic point defect and a region wherein interstitials are the predominant intrinsic point defect, the ingot having at least 2 interstitial dominant regions which are substantially free of agglomerated interstitial defects separated by a vacancy dominant region along the axis of the constant diameter portion of the ingot, wherein the radius of the constant diameter portion of the ingot is at least about 75 mm.

27. An ingot according to claim 26, having a radius of at least about 100 mm or greater, preferably at least about 150 mm.

28. An ingot according to claim 26 or claim 27, wherein the length of the constant diameter portion of the ingot is at least about 400 mm, for example at least about 600 mm, or at least about 800 mm, or at least about 1000 mm.

29. A population of wafers sliced from an ingot having a central axis, a seed-cone, an end-cone, produced by a process according to any one of claims 1 to 28, said wafers being substantially free of agglomerated interstitial defects, wherein the wafers were selected based on the diameter of the wafer.

30. A population of wafers sliced from a single silicon ingot, each wafer being substantially free of agglomerated intrinsic point defects, wherein at least one wafer has crystal lattice vacancies as the predominant intrinsic point defect throughout the wafer, and at least one wafer has silicon self-interstitials as the predominant intrinsic point defect throughout the wafer.

**Patentansprüche**

1. Verfahren zur Herstellung eines Silizium-Einkristalls, bei dem geschmolzenes Silizium nach der Czochralski-Methode zu einem Kristall unter Bildung eines Blocks erstarrt, der eine Mittelachse, einen Keimkonus, einen Endkonus, zwischen dem Keimkonus und dem Endkonus einen Teil von konstantem Durchmesser mit einer Seitenfläche, und einen sich von der Mittelachse zu der Seitenfläche erstreckenden Radius R von wenigstens etwa 75 mm hat, wobei der Teil von konstantem Durchmesser eine achsiale Länge L hat, bei dem man

während des Wachstums des Kristallteils von konstantem Durchmesser ein Verhältnis $v/G_o$ steuert, wobei v eine Wachstumsgeschwindigkeit und $G_o$ ein mittlerer achsialer Temperaturgradient über den Temperaturbereich von der Erstarrung bis zu einer Temperatur von nicht weniger als etwa 1325 °C ist, um zu Anfang in dem Blockteil von konstantem Durchmesser eine Reihe längs der Achse alternierender vorherrschender Eigenpunktfehlstellen zu bilden, die $N_{vac.}$ Bereiche mit vorherrschenden Leerstellen und $N_{int.}$ Bereiche mit vorherrschendem Selbstzwischengitter-Silizium aufweist, wobei $N_{vac.}$ wenigstens 2 und $N_{int.}$ wenigstens 1 ist, die Bereiche mit vorherrschende Leerstellen jeweils eine axiale Länge $L_{vac.}$ und einen Radius $R_{vac.}$ haben, der sich von der Blockachse zu der Seitenfläche erstreckt und wenigstens etwa 10 % des Radius R des Kristallteils von konstantem Durchmesser beträgt, und der Bereich bzw. die Bereiche mit vorherrschendem Selbstzwischengitter-Silizium jeweils eine axiale Länge $L_{int.}$ und eine radiale Breite haben, die gleich dem Radius R des Teils des Silizium-Einkristalls von konstantem Durchmesser ist, und

die genannten Bereiche von der Erstarrungstemperatur mit einer Geschwindigkeit abkühlt, die den Selbstzwischengitter-Siliziumatomen erlaubt, radial zu der Seitenfläche und axial zu den Bereichen mit vorherrschenden Leerstellen zu diffundieren, um die Konzentration der Selbstzwischengitter-Siliziumatome in dem Bereich bzw. den Bereichen zu verringern, in denen die Selbstzwischengitter-Siliziumatome anfangs die vorherrschende Eigenpunktfehlstelle waren, und die Konzentration der Kristallgitter-Leerstellen in dem Bereich bzw. den Bereichen zu verringern, in denen Kristallgitter-Leerstellen anfangs die vorherrschende Eigenpunktfehlstelle waren.

2. Verfahren nach Anspruch 1, bei dem das Verhältnis $v/G_o$ durch Steuerung der Wachstumsgeschwindigkeit v gesteuert wird.

3. Verfahren nach Anspruch 1 oder Anspruch 2, bei dem die Bereiche, in denen die Kristallgitter-Leerstellen die vorherrschenden Eigenpunktfehlstellen sind, einen sich von der Blockachse zur Seitenfläche erstreckenden Radius $R_{vac.}$ haben, der wenigstens etwa 50 % (vorzugsweise wenigstens etwa 90 %) des Radius R des Kristallteils von konstantem Durchmesser beträgt.

4. Verfahren nach einem der Ansprüche 1 bis 3, bei dem die Abkühlungsgeschwindigkeit des Blocks so gesteuert wird, daß die Bereiche von der Erstarrungstemperatur auf eine Temperatur $T_A$ abkühlen, bei der sich Keime agglomerierter Eigenpunktfehlstellen bilden, so daß die Bereiche auf einer Temperatur oberhalb $T_A$ für einen Zeitraum von wenigstens einer wirksamen Verweilzeit $t_{dw\text{-}eff.}$ gehalten werden, die erforderlich ist, um die Bildung agglomerierter Eigenpunktfehlstellen in den Bereichen mit vorherrschenden Zwischengitteratomen zu verhindern.

5. Verfahren nach Anspruch 4, bei dem die Temperatur $T_A$, bei der sich Keime agglomerierter Zwischengitteratome bilden, etwa 850°C bis etwa 1.100°C (vorzugsweise etwa 870°C bis etwa 970°C) beträgt.

6. Verfahren nach Anspruch 2, bei dem die Wachstumsgeschwindigkeit so gesteuert wird, daß die Wachstumsgeschwindigkeit $v_{vac.}$ während der Bildung des Bereiches mit vorherrschenden Leerstellen wenigstens etwa das 1,5-fache eines kritischen Wertes $v_{crit.}$ ist und die Wachstumsgeschwindigkeit $v_{int.}$ während der Bildung des Bereichs mit vorherrschenden Zwischengitteratomen kleiner als das etwa 0,9-fache des kritischen Wertes $v_{crit.}$ ist.

7. Verfahren nach Anspruch 6, bei dem das Verhältnis $L_{vac.}/L_{int.}$ wenigstens etwa 0,23 beträgt.

8. Verfahren nach Anspruch 6 oder Anspruch 7, bei dem $v_{vac.}$ während des Wachstums der Bereiche mit vorherrschenden Leerstellen wenigstens das etwa 2-fache des kritischen Wertes $v_{crit.}$ beträgt.

9. Verfahren nach Anspruch 8, bei dem das Verhältnis $L_{vac.}/L_{int.}$ wenigstens etwa 0,17 beträgt.

10. Verfahren nach Anspruch 6, bei dem $V_{vac.}$ während des Wachstums der Bereiche mit vorherrschenden Leerstellen wenigstens das etwa 2,5-fache des kritischen Wertes $v_{crit.}$ beträgt.

11. Verfahren nach Anspruch 10, bei dem das Verhältnis $L_{vac.}/L_{int.}$ wenigstens etwa 0,14 beträgt.

12. Verfahren nach Anspruch 2, bei dem die Wachstumsgeschwindigkeit so gesteuert wird, daß die Wachstumsgeschwindigkeit $v_{vac.}$ während der Bildung des Bereiches mit vorherrschenden Leerstellen wenigstens das etwa 1,5-fache eines kritischen Wertes $v_{crit.}$ beträgt und die Wachstumsgeschwindigkeit $v_{int.}$ während der Bildung des Bereichs mit vorherrschenden Zwischengitteratomen weniger als das etwa 0,5-fache des kritischen Werts $v_{crit.}$ beträgt.

13. Verfahren nach Anspruch 12, bei dem das Verhältnis $L_{vac.}/L_{int.}$ wenigstens etwa 0,12 beträgt.

14. Verfahren nach Anspruch 12, bei dem $v_{vac.}$ während des Wachstums der Bereiche mit vorherrschenden Leerstellen wenigstens das etwa 2-fache des kritischen Wertes $v_{crit.}$ beträgt.

15. Verfahren nach Anspruch 14, bei dem das Verhältnis $L_{vac.}/L_{int.}$ wenigstens etwa 0,1 beträgt.

16. Verfahren nach Anspruch 12, bei dem $v_{vac.}$ während des Wachstums der Bereiche mit vorherrschen Leerstellen wenigstens das etwa 2,5-fache des kritischen Wertes $v_{crit.}$ beträgt.

17. Verfahren nach Anspruch 16, bei dem das Verhältnis $L_{vac.}/L_{int.}$ wenigstens etwa 0,08 beträgt.

18. Verfahren nach einem der Ansprüche 1 bis 17, bei dem der bzw. die Bereich(e), in dem bzw. denen Selbstzwischengitter-Siliziumatome die vorherrschenden Eigenpunktfehlstellen sind, eine axiale Länfe $L_{int.}$ haben , die wenigstens etwa 25 % des Radius R des Kristallteils von konstantem Durchmesser beträgt, aber vorzugsweise kleiner als etwa der zweifache Radius R des Kristallteils von konstantem Durchmesser ist und noch bevorzugter eine axiale Länge $L_{int.}$ haben, die etwa gleich dem Radius R des Kristallteils von konstantem Durchmesser ist.

19. Verfahren nach Anspruch 18, bei dem in einem Block, in dem Selbstzwischengitter-Siliziumatome die vorherrschenden Eigenpunktfehlstellen in dem gesamten Blockteil von konstantem Durchmesser sind, die wirksame Verweilzeit $t_{dw\text{-}eff.}$ wenigstens etwa 20 % der Verweilzeit ist, die nötig ist, daß eine genügende Menge Selbstzwischengitter-Siliziumatome zu der Blockoberfläche diffundieren kann, um die Konzentration der Selbstzwischengitter-Siliziumatome nur unter eine kritische Konzentration zu drücken, die zur Keimbildung agglomerierter Eigenpunktfehlstellen erforderlich ist.

20. Verfahren nach Anspruch 19, bei dem in einem Block, in dem Selbstzwischengitter-Siliziumatome die vorherrschenden Eigenpunktfehlstellen in dem gesamten Blockteil von konstantem Durchmesser sind, die wirksame Verweilzeit $t_{dw\text{-}eff.}$ weniger als etwa 85 % der Verweilzeit, vorzugsweise etwa 60 % der Verweilzeit ist, die nötig ist, daß eine genügende Menge Selbstzwischengitter-Siliziumatome zu der Blockoberfläche diffundieren kann, um die Konzentration der Selbstzwischengitter-Siliziumatome nur unter eine kritische Konzentration zu drücken, die zur Keimbildung agglomerierter Eigenpunktfehlstellen erforderlich ist.

21. Verfahren nach einem der Ansprüche 1 bis 20, bei dem der Radius R des Blockteils von konstantem Durchmesser wenigstens etwa 100 mm, vorzugsweise wenigstens etwa 150 mm beträgt.

22. Verfahren nach einem der Ansprüche 1 bis 21, bei dem die Länge L des Blockteils von konstantem Durchmesser wenigstens etwa 400 mm, zum Beispiel wenigstens etwa 600 mm oder wenigstens etwa 1000 mm beträgt.

23. Verfahren nach einem der Ansprüche 1 bis 22, bei dem der Blockteil von konstantem Durchmesser anfangs wenigstens etwa 2 oder 4 oder 6 oder 8 Bereiche mit vorherrschenden Leerstellen aufweist.

24. Verfahren nach einem der Ansprüche 1 bis 23, bei dem die Gesamtmenge der Leerstellen in den Anfangsbereichen mit vorherrschenden Leerstellen größer als die Gesamtmenge an Zwischengitteratomen in den Anfangsbereichen mit vorherrschenden Zwischengitteratomen ist.

25. Verfahren nach einem der Ansprüche 1 bis 24, bei dem man ferner den Block einer Abschreckkühlung auf eine Temperatur unterwirft, die kleiner als eine Temperatur ist, bei der sich Keime agglomerierter Eigenpunktfehlstellen bilden.

26. Einkristall-Siliziumblock mit einer Mittelachse, einem Keimkonus, einem Endkonus und einem Teil von konstantem Durchmesser zwischen dem Keimkonus und dem Endkonus mit einem Umfangsrand und einem sich von der Mittelachse zum Umfangsrand erstreckenden Radius, wobei der Einkristall-Siliziumblock nach dem Züchten und Abkühlen von der Erstarrungstemperatur einen Teil von konstantem Durchmesser hat, der viele axialsymmetrische Bereiche aufweist, die entlang der Blockachse zwischen einem Bereich, in dem Leerstellen die vorherrschenden Eigenpunktfehlstellen sind, und einem Bereich, wo Zwischengitteratome die vorherrschenden Eigenpunktfehlstellen sind, alternieren, wobei der Block längs der Achse des Blockteils von konstantem Durchmesser wenigstens 2 Bereiche mit vorherrschenden Zwischengitteratomen hat, die im wesentlichen frei von agglomerierten Zwischengitterfehlstellen sind und durch einen Bereich mit vorherrschenden Leerstellen getrennt sind, wobei der Radius des Blockteils von konstantem Durchmesser wenigstens etwa 75 mm beträgt.

27. Block nach Anspruch 26 mit einem Radius von wenigstens etwa 100 mm oder größer, vorzugsweise wenigstens etwa 150 mm.

28. Block nach Anspruch 26 oder Anspruch 27, bei dem die Länge des Blockteils von konstantem Durchmesser wenigstens etwa 400 mm, z.B. wenigstens etwa 600 mm oder wenigstens etwa 800 mm oder wenigstens etwa 1000 mm beträgt.

29. Gesamtheit von Wafern, die scheibenförmig aus einem Block geschnitten sind, der eine Mittelachse, einen Keimkonus, einen Endkonus hat und durch ein Verfahren nach einem der Ansprüche 1 bis 28 hergestellt wurde, wobei die genannten Wafer im wesentlichen frei von agglomerierten Zwischengitterfehlstellen sind und die Wafer auf Grund des Waferdurchmessers ausgewählt wurden.

30. Gesamtheit von Wafern, die scheibenförmig aus einem einzelnen Siliziumblock geschnitten sind, wobei jeder Wafer im wesentlichen frei von agglomerierten Eigenpunktfehlstellen ist, wobei wenigstens ein Wafer Kristallgitterleerstellen als die vorherrschende Eigenpunktfehlstelle in dem Wafer hat und wenigstens ein Wafer Selbstzwischengitter-Silizium als die vorherrschende Eigenpunktfehlstelle in dem Wafer hat.

## Revendications

1. Procédé pour la préparation d'un monocristal de silicium dans lequel le silicium fondu est solidifié en un cristal conformément au procédé Czochralski pour former un lingot ayant un axe central, un cône de départ, un cône de fin, une partie à diamètre constant entre le cône de départ et le cône de fin ayant une surface latérale, et un rayon, R, s'étendant de l'axe central à la surface latérale d'au moins 75 mm environ, la partie à diamètre constant ayant une longueur axiale, L, le procédé comprenant :

le contrôle d'un rapport, $v/G_0$, où v est une vitesse de croissance et $G_0$ est un gradient de température axial moyen sur la plage de température de la solidification jusqu'à une température d'au moins 1325°C environ, pendant la croissance de la partie à diamètre constant du cristal, pour produire initialement dans la partie à diamètre constant du lingot une série de défauts ponctuels intrinsèques prédominants alternés le long de l'axe, la série comprenant $N_{vac.}$ régions dominées par des lacunes et $N_{int.}$ régions dominées par des auto-interstitiels de silicium, où $N_{vac.}$ est au moins 2 et $N_{int.}$ est au moins 1, les régions dominées par des lacunes ayant chacune une longueur axiale, $L_{vac.}$, et un rayon, $R_{vac.}$, s'étendant de l'axe du lingot vers la surface latérale qui est au moins environ 10 % du rayon, R, de la partie à diamètre constant du cristal, la (les) région(s) dominée(s) par des auto-interstitiels de silicium (6) ayant chacune une longueur axiale, $L_{int.}$, et une largeur radiale qui est égale au rayon, R, de la partie à diamètre constant du monocristal de silicium ;

le refroidissement desdites régions de la température de solidification à une vitesse qui permet aux atomes auto-interstitiels de silicium de se diffuser radialement à la surface latérale et de se diffuser axialement aux régions dominées par des lacunes pour réduire la concentration d'atomes auto-interstitiels de silicium dans la (les) région(s) dans laquelle (lesquelles) des atomes auto-interstitiels de silicium étaient initialement le défaut ponctuel intrinsèque prédominant, et pour réduire la concentration des lacunes de maille cristalline dans la (les) région(s) dans laquelle (lesquelles) des lacunes de maille cristalline étaient initialement le défaut ponctuel intrinsèque prédominant.

2. Procédé selon la revendication 1, dans lequel le rapport, $v/G_0$, est contrôlé en contrôlant la vitesse de croissance, v.

3. Procédé selon la revendication 1 ou la revendication 2, dans lequel les régions dans lesquelles des lacunes de maille cristalline sont les défauts ponctuels intrinsèques prédominants, ont un rayon, $R_{vac.}$, s'étendant de l'axe du lingot vers la surface latérale qui est au moins environ 50 % (de préférence au moins environ 90 %) du rayon, R, de la partie à diamètre constant du cristal.

4. Procédé selon l'une quelconque des revendications 1 à 3, dans lequel la vitesse de refroidissement du lingot est contrôlée de sorte que les régions soient refroidies de la température de solidification à une température, $T_A$, à laquelle les défauts ponctuels intrinsèques forment des germes, de sorte que les régions soient maintenues à une température supérieure à $T_A$ pendant une période de temps d'au moins une durée de maintien effective, $t_{dw-eff.}$, nécessaire pour empêcher la formation de défauts ponctuels intrinsèques agglomérés dans les régions dominées par des interstitiels.

5. Procédé selon la revendication 4, dans lequel la température, $T_A$, à laquelle des interstitiels agglomérés forment des germes, va d'environ 850°C à environ 1100°C (de préférence à partir d'environ 870°C à environ 970°C).

6. Procédé selon la revendication 2, dans lequel la vitesse de croissance est contrôlée de sorte que la vitesse de croissance pendant la formation de la région dominée par des lacunes, $v_{VBC.}$, est au moins environ 1,5 fois une valeur critique, $v_{crit.}$, et la vitesse de croissance pendant la formation de la région dominée par des interstitiels, Vint., est moins d'environ 0,9 fois la valeur critique $v_{crit.}$.

7. Procédé selon la revendication 6, dans lequel le rapport de $L_{vac.}/L_{int.}$ est au moins environ 0,23.

8. Procédé selon la revendication 6 ou la revendication 7, dans lequel $v_{vac.}$ est au moins, environ 2 fois la valeur critique, $v_{crit.}$, pendant la croissance des régions dominées par des lacunes.

9. Procédé selon la revendication 8, dans lequel le rapport de $L_{vac.}/L_{int.}$ est au moins environ 0,17.

10. Procédé selon la revendication 6, dans lequel $v_{vac.}$ est au moins environ 2,5 fois la valeur critique, $v_{crit.}$, pendant la croissance des régions dominées par des lacunes.

11. Procédé selon la revendication 10, dans lequel le rapport de $L_{vac.}/L_{int.}$ est au moins environ 0,14.

12. Procédé selon la revendication 2, dans lequel la vitesse de croissance est contrôlée de sorte que la vitesse de croissance pendant la formation de la région dominée par des lacunes, $v_{vac.}$, est au moins environ 1,5 fois une valeur critique, $v_{crit.}$, et la vitesse de croissance pendant la formation de la région dominée par des interstitiels, $v_{int.}$, est moins d'environ 0,5 fois la valeur critique $v_{crit.}$.

13. Procédé selon la revendication 12, dans lequel le rapport de $L_{vac.}/L_{int.}$ est au moins 0,12 environ.

**14.** Procédé selon la revendication 12, dans lequel $v_{vac.}$ est au moins environ 2 fois la valeur critique, $v_{crit.}$, pendant la croissance des régions dominées par des lacunes.

**15.** Procédé selon la revendication 14, dans lequel le rapport de $L_{vac.}/L_{int.}$ est au moins environ 0,1.

**16.** Procédé selon la revendication 12, dans lequel $v_{vac.}$ est au moins environ 2,5 fois la valeur critique, $v_{crit.}$, pendant la croissance des régions dominées par des lacunes.

**17.** Procédé selon la revendication 16, dans lequel le rapport de $L_{vac.}/L_{int.}$ est au moins environ 0,08.

**18.** Procédé selon l'une quelconque des revendications 1 à 17, dans lequel la (les) région(s) dans laquelle (lesquelles) des atomes auto-interstitiels de silicium sont les défauts ponctuels intrinsèques prédominants ont une longueur axiale, $L_{int.}$, qui est au moins environ 25 % du rayon, R, de la partie à diamètre constant du cristal, mais de préférence de moins de deux fois le rayon, R, de la partie à diamètre constant du cristal, et même plus de préférence une longueur axiale, $L_{int.}$, qui est environ égale au rayon, R, de la partie à diamètre constant du cristal.

**19.** Procédé selon la revendication 18, dans lequel la durée de maintien effective, $t_{dw-eff.}$, est au moins environ 20 % de la durée de maintien nécessaire pour permettre à une quantité suffisante d'atomes auto-interstitiels de silicium de se diffuser à la surface du lingot seulement pour supprimer la concentration d'atomes auto-interstitiels de silicium en dessous d'une concentration critique nécessaire pour que les défauts ponctuels intrinsèques agglomérés germent, dans un lingot où des atomes auto-interstitiels de silicium sont le défaut ponctuel intrinsèque prédominant dans toute la partie à diamètre constant d'un lingot.

**20.** Procédé selon la revendication 19, dans lequel la durée de maintien effective, $t_{dw-eff.}$, est inférieure à environ 85 % de la durée de maintien, de préférence environ 60 % de la durée de temporisation, nécessaire pour permettre à une quantité suffisante d'atomes auto-interstitiels de silicium de se diffuser à la surface du lingot seulement pour supprimer la concentration d'atomes auto-interstitiels de silicium en dessous d'une concentration critique nécessaire pour que les défauts ponctuels intrinsèques agglomérés germent, dans un lingot dans lequel des atomes auto-interstitiels de silicium sont le défaut ponctuel intrinsèque prédominant dans toute la partie à diamètre constant d'un lingot.

**21.** Procédé selon l'une quelconque des revendications 1 à 20, dans lequel le rayon de la partie à diamètre constant du lingot, R, est au moins 100 mm environ, de préférence au moins 150 mm environ.

**22.** Procédé selon l'une quelconque des revendications 1 à 21, dans lequel la longueur de la partie à diamètre constant du lingot, L, est au moins 400 mm environ, par exemple, au moins 600 mm environ, ou au moins 1000 mm environ.

**23.** Procédé selon l'une quelconque des revendications 1 à 22, dans lequel la partie à diamètre constant du lingot comprend initialement au moins environ 2 ou 4 ou 6 ou 8 régions dominées par des lacunes.

**24.** Procédé selon l'une quelconque des revendications 1 à 23, dans lequel la quantité totale de lacunes dans les régions initiales dominées par des lacunes est supérieure à la quantité totale d'interstitiels dans les régions initiales dominées par des interstitiels.

**25.** Procédé selon l'une quelconque des revendications 1 à 24, comprenant en outre le refroidissement par étouffage du lingot à une température inférieure à une température à laquelle germent des défauts ponctuels intrinsèques agglomérés.

**26.** Lingot de silicium monocristallin ayant un axe central, un cône de départ, un cône de fin et une partie à diamètre constant entre le cône de départ et le cône de fin ayant un bord périphérique et un rayon s'étendant de l'axe central au bord périphérique, le lingot de silicium monocristallin, après avoir été développé et refroidi à partir de la température de solidification, ayant une partie à diamètre constant comprenant de multiples régions axialement symétriques alternées le long de l'axe du lingot entre une région dans laquelle les lacunes sont le défaut ponctuel intrinsèque prédominant et une région dans laquelle les interstitiels sont le défaut ponctuel intrinsèque prédominant, le lingot ayant au moins 2 régions dominées par des interstitiels, qui sont sensiblement exemptes de défauts interstitiels agglomérés, séparées par une région dominée par des lacunes le long de l'axe de la partie à diamètre constant du lingot, dans laquelle le rayon de la partie à diamètre constant du lingot est au moins 75 mm environ.

27. Lingot selon la revendication 26, ayant un rayon d'au moins 100 mm environ ou supérieur, de préférence d'au moins 150 mm environ.

28. Lingot selon la revendication 26 ou la revendication 27, dans lequel la longueur de la partie à diamètre constant du lingot est au moins 400 mm environ, par exemple au moins 600 mm environ, ou au moins 800 mm environ, ou au moins 1000 mm environ.

29. Population de tranches coupées d'un lingot ayant un axe central, un cône de départ, un cône de fin, produit par un procédé selon l'une quelconque des revendications 1 à 28, lesdites tranches étant sensiblement sans défauts d'interstitiels agglomérés, dans laquelle les tranches ont été choisies sur la base du diamètre de la tranche.

30. Population de plaquettes coupées à partir d'un seul lingot de silicium, chaque tranche étant sensiblement exempte de défauts ponctuels intrinsèques agglomérés, dans laquelle au moins une tranche a des lacunes de maille cristalline comme défaut ponctuel intrinsèque prédominant sur toute la tranche, et au moins une tranche a des auto-interstitiels de silicium comme défaut ponctuel intrinsèque prédominant sur toute la tranche.

# FIG. 1

# FIG. 2

# FIG. 3

Distance Along the Axis of the Ingot

~0 mm
~150 mm
~310 mm
~470 mm
~580 mm
~680 mm
~800 mm

Nucleation Front

## FIG. 4A

No Heat Treat
+ B-defect test

## FIG. 4B

1250C, 10s
+ B-defect test